# EUROPEAN PATENT SPECIFICATION

(11) **EP 2 979 657 B1**
(45) Date of publication and mention of the grant of the patent: **04.07.2018**
(21) Application number: 14775052.5
(22) Date of filing: 14.01.2014
(51) Int. Cl.: A61B 17/00, A61B 90/00, A61B 34/00, A61B 1/00, A61B 18/14, A61B 18/00, A61B 34/30, A61B 34/37, A61B 17/29

(54) **MEDICAL INSTRUMENT AND MEDICAL SYSTEM**
MEDIZINISCHES INSTRUMENT UND MEDIZINISCHES SYSTEM
INSTRUMENT MÉDICAL ET SYSTÈME MÉDICAL

(30) Priority: 28.03.2013 US 201361806101 P
(43) Date of publication of application: 03.02.2016
(73) Proprietor: Olympus Corporation, Tokyo 192-8507 (JP)
(72) Inventor: HYODO, Ryoji, Hachioji-shi, Tokyo 192-8507 (JP); KISHI, Kosuke, Hachioji-shi, Tokyo 192-8507 (JP)
(74) Representative: Gunzelmann, Rainer
(86) International application number: PCT/JP2014/050414
(87) International publication number: WO 2014/156221

(56) References cited:
- EP-A2- 2 014 218
- JP-A- 2008 264 253
- JP-U- H0 225 255
- US-A1- 2006 178 657
- US-A1- 2008 255 422
- US-A1- 2008 312 652
- US-A1- 2010 022 837
- US-A1- 2011 213 360

## Description

### Technical Field

The present invention relates to a medical instrument and a medical system.

### Background Art

Conventionally, in the field of medical instruments used with endoscopes, a medical instrument provided with a storage part in which a treatment part that performs treatment on a treatment target portion is stored in order to protect an inner surface of a channel of the endoscope has been known (for example, see

### Patent Literature 1).

Patent Literature 2 discloses a medical instrument including an insertion part which has a bending part capable of bending in a predetermined range; a plurality of joint rings which are provided in the bending part and are connected to each other in the axial direction of the insertion part; a manipulating part; and a wire which is inserted into the plurality of the joint rings and connects the bending part with the manipulating part, the plurality of the joint rings comprising a connecting part, a portion of which is provided so as to protrude from the outer peripheral surface and which is connected to the joint ring of the distal side in the axial direction of the insertion part and a connected part which is connected to the joint ring of the proximal side in the axial direction of the insertion part.

### Citation List

### Patent Literature

Patent Literature 1: Japanese Unexamined Patent Application, First Publication No. 2008-264253
Patent Literature 2: US Patent Application Publication No. 2008/255422 A1

### Summary of Invention

### Technical Problem

Such a medical instrument may have an electrode that protrudes toward a distal end thereof in a needle shape, a rod shape, or a hook shape, or forceps that protrude toward a distal end thereof as a treatment part. When inserted into a channel of a manipulator such as an endoscope, this medical instrument may damage an inner surface of the channel or interfere with the inner surface of the channel and be unable to smoothly pass through the channel. For this reason, it has conventionally been known that a cover member is provided at an outer portion of the electrode or the forceps and a mechanism that draws the electrode or the forceps into the cover member is provided on the medical instrument.

However, when the cover member is provided, the cover member covers the electrode or the forceps, and thus an outer diameter thereof is easily increased. When the electrode or the forceps are drawn into the cover member, a mechanism for moving the cover member relative to the electrode or the forceps needs to be separately provided for the medical instrument.

An object of the present invention is to miniaturize a medical instrument equipped with a mechanism that changes a direction or orientation of a treatment part and a mechanism that stores the treatment part.

### Solution to Problem

The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes. According to a first aspect of the present invention, a medical instrument includes the features of claim 1. A medical instrument may include: an insertion part configured to be inserted into a human body; and a driving mechanism coupled to the insertion part. The insertion part has: a treatment part configured to perform treatment on a treatment target portion; a joint part configured to support the treatment part and to be capable of changing a direction of the treatment part; and a driving force transmission part connected to the joint part and configured to transmit a driving force that changes the direction of the treatment part to the joint part. The driving mechanism has a driving force generator that is connected to the driving force transmission part and is configured to generate the driving force. A storage part configured to store the treatment part in the joint part by the driving force transmitted from the driving force generator to the joint part via the driving force transmission part is formed at the joint part.

According to a second aspect of the present invention, in the medical instrument according to the first aspect of the present invention, the joint part may have a flexible spring part that is bendably configured to change the direction of the treatment part and has a substantially cylindrical shape. The driving force transmission part may have a plurality of linear members that are provided corresponding to a bending direction in bending movement of the joint part and are fixed to a distal end of the flexible spring part. The flexible spring part may be bent by at least one of the plurality of linear members being pulled. At least two of the plurality of linear members may be uniformly pulled or pressed such that the distal end of the flexible spring part advances or retracts in a center axis direction of the flexible spring part, and thereby the storage part may be formed adjacent to the distal end of the flexible spring part.

According to a third aspect of the present invention, the medical instrument according to the second aspect of the present invention may further include a cladding tube part configured to cover an outer surface of the flexible spring part. The distal end of the flexible spring part may be retracted toward a proximal end side of the flexible spring part by the plurality of linear members, and thereby the storage part may be formed in the cladding tube part.

According to a fourth aspect of the present invention, in the medical instrument according to the second aspect of the present invention, the treatment part may be inserted into the flexible spring part. The distal end of the flexible spring part may be advanced toward a distal end side of the flexible spring part by the plurality of linear members, and thereby the storage part may be formed in the flexible spring part.

According to a fifth aspect of the present invention, in the medical instrument according to any one of the first aspect to the fourth aspect of the present invention, the treatment part may have an incision electrode configured to receive supply of a high-frequency current to incise biological tissue. The joint part may have a switching mechanism configured to switch a conductive state of the high-frequency current for the incision electrode. The insertion part may have a power-supplying cable that is connected to the switching mechanism and is configured such that the high-frequency current is applied. The driving mechanism may have a plug that is connected to the power-supplying cable and is configured to be connectable to a high-frequency power supply. The switching mechanism may interrupt conduction between the power-supplying cable and the incision electrode when the incision electrode is inside the storage part, and may connect the power-supplying cable and the incision electrode when the incision electrode is outside the storage part.

According to a sixth aspect of the present invention, a medical system includes: the medical instrument according to any one of the first aspect to the fifth aspect of the present invention; a master manipulator configured to receive a manipulation input from an operator; a controller connected to the master manipulator; a slave manipulator connected to the controller and the driving mechanism; and a determination unit provided in the controller and configured to determine a state in which the treatment part is inside the storage part and a state in which the treatment part is outside the storage part. The medical instrument has a switch mechanism configured to cause the determination unit to determine the state in which the treatment part is inside the storage part and the state in which the treatment part is outside the storage part.

### Advantageous Effects of Invention

According to the medical instrument described above, both a mechanism that optimizes a direction and orientation of the treatment part and a mechanism that displaces the treatment part in order to store the treatment part in the storage part can be used in common. For this reason, in comparison with a medical instrument that has a separate constitution specialized for storing the treatment part, the medical instrument can be miniaturized.

### Brief Description of Drawings

Fig. 1 is a schematic view illustrating a medical instrument according to a first embodiment of the present invention.
Fig. 2 is a schematic view illustrating a state in which the medical instrument is mounted in an endoscope.
Fig. 3 is a partial sectional view illustrating an enlarged distal end portion of the medical instrument.
Fig. 4 is a partial sectional view illustrating the enlarged distal end portion of the medical instrument.
Fig. 5 is a view for describing an operation of the medical instrument.
Fig. 6 is a view for describing the operation of the medical instrument.
Fig. 7 is a view for describing the operation of the medical instrument.
Fig. 8 is a schematic side view illustrating an enlarged distal end portion of a medical instrument according to a second embodiment of the present invention.
Fig. 9 is a view for describing an operation of the medical instrument.
Fig. 10 is a view for describing the operation of the medical instrument.
Fig. 11 is a schematic overall view illustrating a medical system according to a third embodiment of the present invention.
Fig. 12 is a schematic partial sectional view illustrating a medical instrument according to a third embodiment of the present invention, wherein the medical instrument is provided in the medical system.
Fig. 13 is a view for describing an operation of the medical system.

### Description of Embodiments

### (First embodiment)

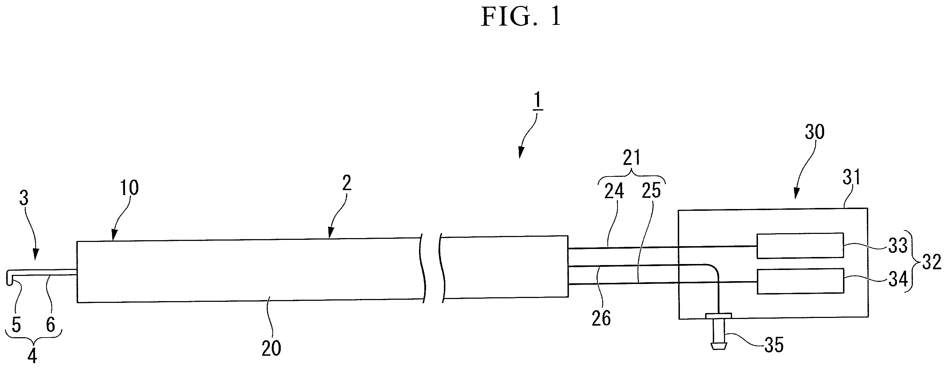

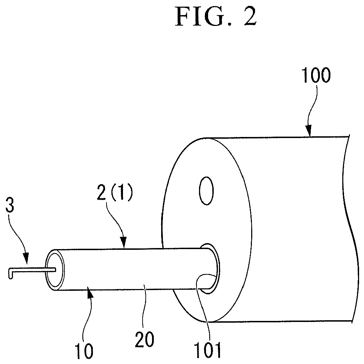

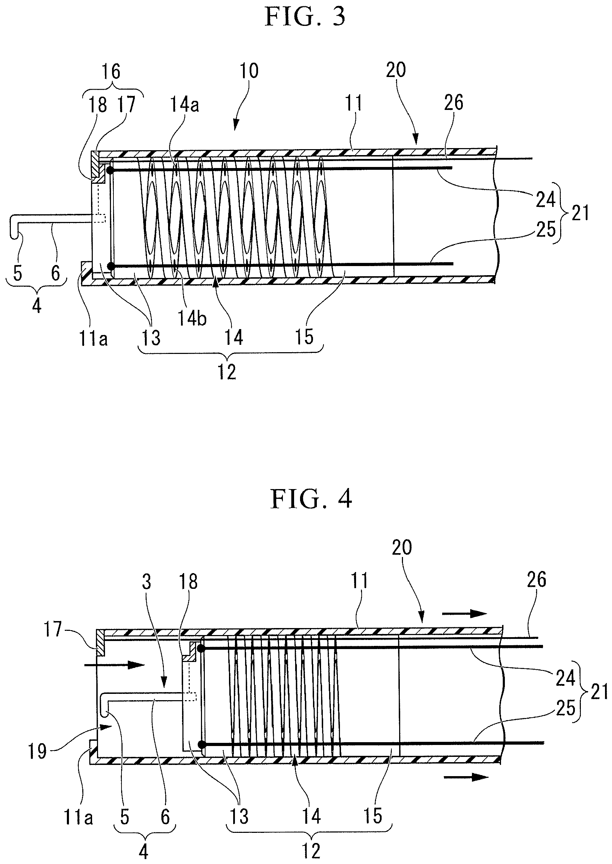

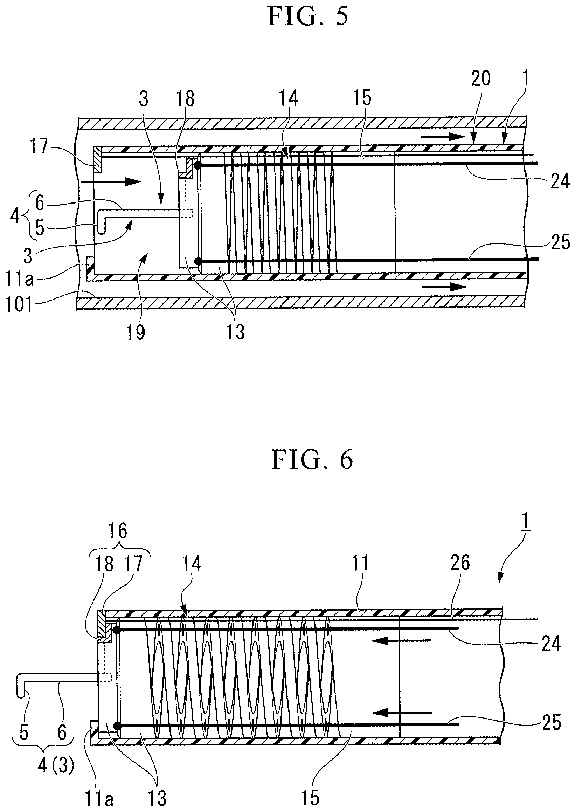

A medical instrument (medical device) according to a first embodiment of the present invention will be described. Fig. 1 is a schematic view illustrating a medical instrument according to the first embodiment of the present invention. Fig. 2 is a schematic view illustrating a state in which the medical instrument is mounted in an endoscope. Fig. 3 is a partial sectional view illustrating an enlarged distal end portion of the medical instrument. Fig. 4 is a partial sectional view illustrating the enlarged distal end portion of the medical instrument. Figs. 5 to 7 are views for describing an operation of the medical instrument.

As illustrated in Fig. 1, the medical instrument 1 according to the present embodiment includes an insertion part 2 that can be inserted into a human body, and a driving mechanism 30 coupled to the insertion part 2.

The insertion part 2 has an elongated shape. The insertion part 2 can be inserted into, for instance, a channel 101 of an endoscope 100 (see Fig. 2) or another known manipulator. As illustrated in Fig. 1, the insertion part 2 includes a treatment part 3, a joint part 10, a flexible tube part 20, a driving force transmission part 21, and a power-supplying cable 26. Hereinafter, a side at which the treatment part 3 of the insertion part 2 is provided is referred to as a distal end, and a side opposite to the side at which the treatment part 3 is provided is referred to as a proximal end.

The treatment part 3 performs treatment on a treatment target portion. The treatment part 3 according to the present embodiment has an incision electrode 4 that is supplied with a high-frequency current and incises biological tissue.

The incision electrode 4 has a rod-shaped shaft part 6 and a hook part 5 provided at a distal end of the shaft part 6. The shaft part 6 and the hook part 5 are made of a metal wire having conductivity. The metal wire serving as the incision electrode 4 has a shape bent at a boundary portion between the shaft part 6 and the hook part 5. An insulating film coating an outer surface of the metal wire may be provided on the shaft part 6. The hook part 5 of the incision electrode 4 may be formed, for instance, in an arc shape. The incision electrode 4 may not have the hook part 5.

As illustrated in Fig. 3, the joint part 10 has a cladding tube part 11, a bending tube 12, and a switching mechanism 16. The cladding tube part 11 is formed by a flexible tubular member, which constitutes the flexible tube part 20, extending to a distal end side thereof. The bending tube 12 is arranged inside the cladding tube part 11, and supports the incision electrode 4. The switching mechanism 16 switches a state in which a high-frequency current is applied to the incision electrode 4.

The distal end of the cladding tube part 11 is provided with a stopper 11a and a terminal 17. The stopper 11a regulates a position of a distal end of the bending tube 12. The terminal 17 is connected to the power-supplying cable 26 to apply a high-frequency current, and constitutes a part of the switching mechanism 16.

The stopper 11a can abut the distal end of the bending tube 12. The stopper 11a regulates movement of the distal end portion of the bending tube 12 such that the distal end of the bending tube 12 does not protrude from the distal end of the cladding tube part 11.

The bending tube 12 has a substantially columnar shape. The bending tube 12 has a distal end support part 13, a flexible spring part 14, and a proximal end support part 15. The distal end support part 13, the flexible spring part 14, and the proximal end support part 15 of the bending tube 12 are arranged in this order from the distal end toward a proximal end of the bending tube 12.

The distal end support part 13 is fixed to a proximal end of the shaft part 6 of the incision electrode 4. Distal ends of first and second wires (linear members) 24 and 25 to be described below are fixed to the distal end support part 13. The distal end support part 13 has an outer surface shaped of a columnar surface. A clearance is formed between the outer surface of the distal end support part 13 and an inner surface of the cladding tube part 11 to such an extent that the distal end support part 13 can slide relative to the cladding tube part 11. A terminal 18 is provided on a surface of a distal end side of the distal end support part 13. The terminal 18 constitutes a part of the switching mechanism 16. The terminal 18 is made of a conductor, and is electrically connected to the incision electrode 4.

The flexible spring part 14 is an elastic member having a helical shape. The flexible spring part 14 has a substantially cylindrical shape. The flexible spring part 14 is coiled inside the cladding tube part 11 along the inner surface of the cladding tube part 11 from a distal end toward a proximal end thereof in a helical shape. The flexible spring part 14 is formed with through-holes 14a and 14b at positions that face each other in a radial direction of the flexible spring part 14. The first wire 24 is inserted into the through-hole 14a. The second wire 25 is inserted into the through-hole 14b. In addition to the first and second wires 24 and 25, a wire configured to bend the joint part 10 may be provided in the medical instrument 1. In this case, in addition to the two through-holes 14a and 14b, other through-holes may be formed at any positions of the flexible spring part 14.

The proximal end support part 15 is fixed to the inner surface of the cladding tube part 11. The proximal end support part 15 is provided to bias the flexible spring part 14 toward a distal end side thereof. The proximal end support part 15 is formed with a through-hole (not shown) into which the first wire 24 is inserted and another through-hole (not shown) into which the second wire 25 is inserted at positions that face each other in a radial direction of the proximal end support part 15.

In the bending tube 12 having the above constitution, the proximal end support part 15 is fixed to the inner surface of the cladding tube part 11. The flexible spring part 14 biases the distal end support part 13 toward the distal end side of the distal end support part 13. The distal end support part 13 biased toward the distal end side thereof by the flexible spring part 14 has a distal end coming into contact with the stopper 11a of the cladding tube part 11. When the distal end support part 13 is in contact with the stopper 11a, the terminals 17 and 18 of the switching mechanism 16 are in contact with each other. If the distal end support part 13 is separated from the stopper 11a toward the proximal end side thereof, the contact between the terminals 17 and 18 of the switching mechanism 16 is released (see Fig. 4).

As illustrated in Fig. 4, the interior of the cladding tube part 11 at the joint part 10 is a storage part 19 that stores the incision electrode 4 of the treatment part 3. That is, as the distal end support part 13 moves toward the proximal end side thereof in the cladding tube part 11, the storage part 19 that stores the incision electrode 4 is formed in the cladding tube part 11. An amount of the incision electrode 4 that is drawn into the storage part 19 is set to such an extent that the incision electrode 4 does not interfere with an inner surface of the channel 101, for instance, when the channel 101 of the endoscope 100 is in a curved state. The joint part 10 may be configured such that the incision electrode 4 is completely drawn into the storage part 19.

The flexible tube part 20 illustrated in Fig. 1 is a flexible tubular member. The first and second wires 24 and 25 constituting the driving force transmission part 21, and the power-supplying cable 26 are inserted into the flexible tube part 20.

As illustrated in Figs. 3 and 4, the driving force transmission part 21 has the first wire 24 and the second wire 25. A distal end of the first wire 24 is fixed to the distal end support part 13. A distal end of the second wire 25 is fixed to the distal end support part 13.

A linear member made of a known material such as stainless steel or a resin may be adequately employed for the first wire 24. The first wire 24 is not limited to a so-called wire, and may be, for instance, a cable. A wire made of a known material such as stainless steel or a resin may be adequately employed for the second wire 25. The first wire 24 and the second wire 25 may be made of materials identical to each other or materials different from each other. The second wire 25 is not limited to a so-called wire, and may be, for instance, a cable.

As illustrated in Fig. 1, proximal ends of the first and second wires 24 and 25 are led into the driving mechanism 30 through the interior of the flexible tube part 20 of the insertion part 2. The proximal ends of the first and second wires 24 and 25 are connected to a driving force generator 32. The driving force generator 32 generates a driving force for displacing each of the first wire 24 and the second wire 25 in its center line direction. Although details will be described below, the driving force generated by the driving force generator 32 is a driving force for bending the joint part 10 in order to change the direction of the treatment part 3 as well as a driving force for drawing the incision electrode 4 of the treatment part 3 into the storage part 19.

The power-supplying cable 26 illustrated in Fig. 1 is a flexible wire having a conductive core to which a high-frequency current flows, and an insulating film coating the core. As illustrated in Fig. 3, a distal end of the power-supplying cable 26 is connected to the terminal 17 disposed at the distal end of the cladding tube part 11. As illustrated in Fig. 1, a proximal end of the power-supplying cable 26 is fixed to a plug 35 (to be described below) in the driving mechanism 30. Thereby, according to the medical instrument 1 of the present embodiment, when the terminals 17 and 18 are in contact with each other, the high-frequency current can be supplied from the plug 35 to the incision electrode 4 through the power-supplying cable 26.

As illustrated in Fig. 1, the driving mechanism 30 includes a base 31, the driving force generator 32, and the plug 35.

The driving force generator 32 has a first actuator 33 and a second actuator 34. The first actuator 33 generates a driving force pulling the first wire 24. The second actuator 34 generates a driving force pulling the second wire 25. Specific constitutions of the first and second actuators 33 and 34 are not restricted as long as a suitable traction force is transmitted to the first and second wires 24 and 25. The first actuator 33 and the second actuator 34 can be operated independently of each other. That is, the first actuator 33 can pull the first wire 24, and the second actuator 34 releases the traction force for the second wire 25 so that the second wire 25 can be freely operated. Also, the first actuator 33 releases the traction force for the first wire 24 so that the first wire 24 can be freely operated, and the second actuator 34 can pull the second wire 25. The first wire 24 and the second wire 25 can also be simultaneously pulled by the first actuator 33 and the second actuator 34. As a preferred example of the driving force generator 32, a mechanism that converts a driving force of a motor into linear motion using a rack and pinion and pulls the first wire 24 and the second wire 25 or a mechanism that is provided with pulleys around which the first wire 24 and the second wire 25 are wound and rotates these pulleys using respective motors may be used for the first actuator 33 and the second actuator 34.

The plug 35 is made of a conductor, and can be connected to a known high-frequency power supply. The plug 35 is connected to the proximal end of the power-supplying cable 26.

Next, an operation of the medical instrument 1 according to the present embodiment will be described.

The medical instrument 1 is, for instance, inserted into the channel 101 provided in the manipulator such as the endoscope 100 illustrated in Fig. 2. The channel 101 is provided to guide forceps and other instruments up to a treatment target portion.

When the medical instrument 1 is inserted into the channel 101, the first wire 24 and the second wire 25 are pulled by the driving force generator 32 such that the treatment part 3 moves into the storage part 19. At this time, the first and second actuators 33 and 34 pull the first and second wires 24 and 25 toward the proximal end sides of the first and second wires 24 and 25 at the same time. Thereby, as illustrated in Fig. 5, the distal end support part 13 moves toward the proximal end side thereof along the central axis of the cladding tube part 11 to undergo parallel movement. Accordingly, the distal end support part 13 moves toward the proximal end side thereof relative to the cladding tube part 11 without being locked on the cladding tube part 11. Thereby, the flexible spring part 14 is contracted, and the storage part 19 that stores the incision electrode 4 in the cladding tube part 11 is formed in the cladding tube part 11. For this reason, when the channel 101 is bent, the treatment part 3 is unlikely to interfere with the inner surface of the channel 101, and the treatment part 3 is also unlikely to damage the inner surface of the channel 101.

In a state in which the treatment part 3 is stored in the storage part 19, the treatment part 3 is guided up to the vicinity of the treatment target portion. In a state in which the treatment part 3 and the joint part 10 are fed from a distal end of the channel 101, when a treatment using the treatment part 3 is performed on a treatment target portion, the treatment part 3 is displaced out of the storage part 19.

To displace the treatment part 3 out of the storage part 19, as illustrated in Fig. 6, in the present embodiment, traction of the first wire 24 and traction of the second wire 25 are released by the first actuator 33 and the second actuator 34, respectively. Thereby, the flexible spring part 14 that is in a contracted state is expanded, and the incision electrode 4 is fed from the distal end of the cladding tube part 11. When the distal end of the distal end support part 13 comes into contact with the stopper 11a of the distal end of the cladding tube part 11, the terminal 17 and the terminal 18 come in electrical contact with each other. Thereby, a high-frequency current can be supplied to the incision electrode 4.

When treatment of the treatment target portion is performed, the joint part 10 is bent, and thereby the direction and orientation of the incision electrode 4 can be changed. The joint part 10 is bent by the operations of the first and second actuators 33 and 34.

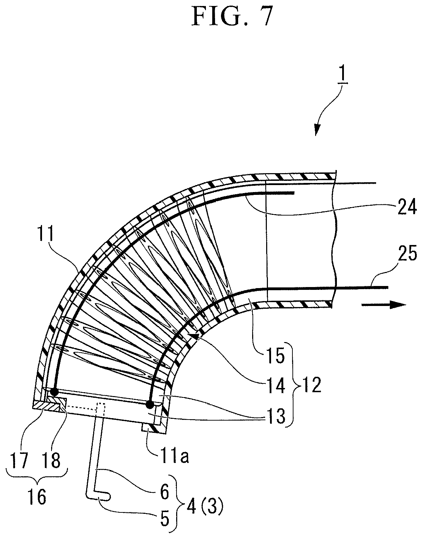

That is, when the joint part 10 is bent, for example, only one of the first actuator 33 and the second actuator 34 pulls the wire as illustrated in Fig. 7. When only one of the first actuator 33 and the second actuator 34 performs the pulling movement, only one of the first wire 24 and the second wire 25 is pulled toward the proximal end side thereof. For example, when only the second wire 25 of the first and second wires 24 and 25 is pulled, a portion of the flexible spring part 14 at which the second wire 25 is disposed is contracted. In contrast, when only the second wire 25 is pulled, the first wire 24 is not pulled. Thus, a portion of the flexible spring part 14 at which the first wire 24 is disposed is not contracted, or may be expanded. Thereby, when only the second wire 25 of the first and second wires 24 and 25 is pulled, the flexible spring part 14 is bent toward the side of the second wire 25.

When any one of the first and second wires 24 and 25 is pulled toward the proximal end side thereof, the distal end support part 13 is very slightly inclined, and is locked on the inner surface of the cladding tube part 11 by friction. This frictional force is applied to the distal end support part 13 and the cladding tube part 11 such that the distal end support part 13 is restrained from moving toward the proximal end side of the cladding tube part 11 relative to the cladding tube part 11. Thereby, the cladding tube part 11 is bent depending on the bending movement of the flexible spring part 14. Thereby, the joint part 10 is bent as a whole.

When only one of the first actuator 33 and the second actuator 34 performs the pulling movement, the other of the first actuator 33 and the second actuator 34 may press the wire connected thereto toward the distal end side of the connected wire. In this case, it is easy to bend the joint part 10 regardless of the magnitude of friction between the distal end support part 13 and the inner surface of the cladding tube part 11. However, when the first wire 24 and the second wire 25 have flexible constitutions liable to undergo buckling due to pressing, it may be preferable for the first and second actuators 33 and 34 not to press the first and second wires 24 and 25 toward the distal end sides of the first and second wires 24 and 25.

In the present embodiment, since the high-frequency current is supplied to the incision electrode 4 of the treatment part 3 through the power-supplying cable 26, the treatment target portion can be incised.

When treatment such as an incision of the treatment target portion is completed or the medical instrument 1 is replaced, the first and second wires 24 and 25 are pulled toward the proximal end sides thereof together, and thereby the treatment part 3 is stored in the storage part 19 again. Thereby, the treatment part 3 does not come in contact with the inner surface of the channel 101, and the medical instrument 1 can be removed from the channel 101.

In the present embodiment, when only one of the first wire 24 and the second wire 25 is pulled, the joint part 10 is bent. When both the first wire 24 and the second wire 25 are pulled at the same time, the storage part 19 is formed in the cladding tube part 11 of the joint part 10, and the incision electrode 4 is stored in the storage part 19. That is, according to the medical instrument 1 of the present embodiment, when treatment of the treatment target portion is performed using the treatment part 3, both the mechanism that optimizes the direction and orientation of the treatment part 3 and the mechanism that displaces the treatment part 3 to store the treatment part 3 in the storage part 19 can be used in common. Accordingly, in comparison with a medical instrument that has a separate constitution specialized for storing the treatment part 3, the medical instrument 1 can be miniaturized. Thereby, it is possible to easily pass the insertion part 2 of the medical instrument 1 into the channel 101 that is in a curved state.

The medical instrument 1 according to the present embodiment has a constitution in which the storage part 19 is formed at a part of the joint part 10 to be curved. For this reason, in comparison with when a cover member or the like that stores the treatment part 3 is provided at the distal end of the joint part 10, a longitudinal dimension of the insertion part 2 can be shortened.

### (Second embodiment)

Next, a medical instrument according to a second embodiment of the present invention will be described. Hereinafter, the same components as those of the embodiment that has already been described are given the same reference signs, and duplicate description thereof will be omitted.

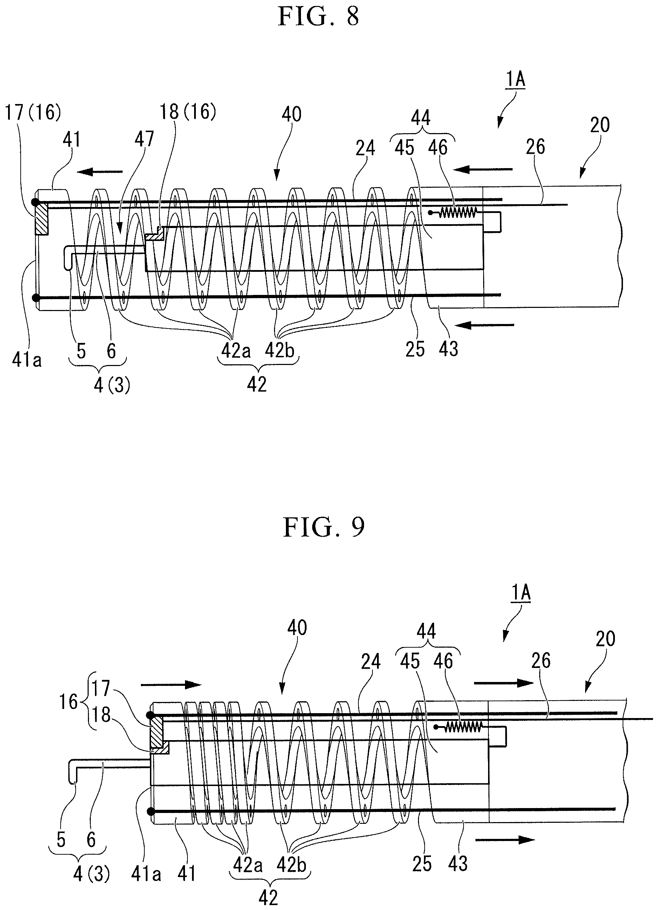

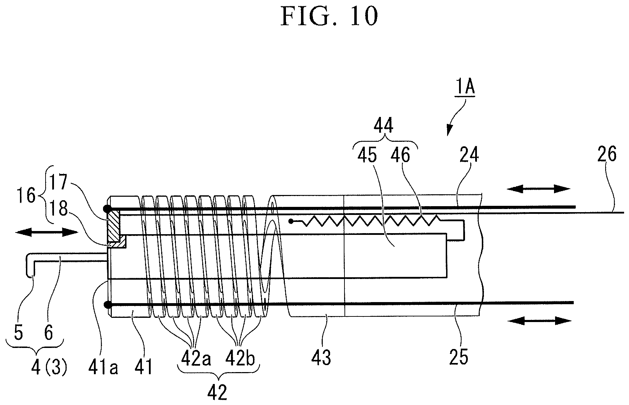

Fig. 8 is a schematic side view illustrating an enlarged distal end portion of a medical instrument according to the second embodiment of the present invention. Figs. 9 and 10 are views for describing an operation of the medical instrument.

As illustrated in Fig. 8, in place of the flexible tube part 20 described in the first embodiment, the medical instrument 1A according to the present embodiment has an expansion pipe part 40 whose structure is different from that of the flexible tube part 20.

The expansion pipe part 40 has a substantially cylindrical shape. The expansion pipe part 40 has a distal end support part 41, a flexible spring part 42, and a proximal end support part 43. The distal end support part 41, the flexible spring part 42, and the proximal end support part 43 of the expansion pipe part 40 are arranged in this order from a distal end toward a proximal end of the expansion pipe part 40.

The distal end support part 41 has a cylindrical shape. The distal end support part 41 has a space into which an incision electrode 4 can be inserted. The terminal 17 of the switching mechanism 16 described in the first embodiment is arranged on an inner surface of the distal end support part 41. The distal end support part 41 has a stopper 41a engaged with a distal end of a shaft extension part 45 (to be described below). When the distal end support part 41 is engaged with the shaft extension part 45, the stopper 41a keeps the terminal 17 and the terminal 18 of the switching mechanism 16 in contact with each other. A distal end of a first wire 24 and a distal end of a second wire 25 are fixed to the distal end support part 41.

The flexible spring part 42 is an elastic member having a helical shape. The flexible spring part 42 is coiled from a distal end toward a proximal end thereof in a helical shape. As in the first embodiment, the flexible spring part 42 is formed with through-holes into which the first wire 24 and the second wire 25 are inserted. The flexible spring part 42 has a flexible spring section 42a disposed at the distal end side thereof and a rigid spring section 42b disposed at the proximal end side thereof.

In the present embodiment, the flexible spring section 42a is a spring member. When no external force is applied to the flexible spring section 42a, wires (element wires) constituting the flexible spring section 42a are separated from one another. The flexible spring section 42a is configured such that, as the first wire 24 and the second wire 25 are pulled toward the proximal end sides thereof at the same time, the element wires come into close contact with one another.

The rigid spring section 42b is a spring member. The rigid spring section 42b is configured such that, even in a step in which the element wires of the flexible spring section 42a come into close contact, a gap is present between element wires constituting the rigid spring section 42b.

For example, in the flexible spring part 42, a spring constant of the rigid spring section 42b is greater than that of the flexible spring section 42a.

The proximal end support part 43 is fixed to a distal end of the flexible tube part 20. The proximal end support part 43 is provided with a holding part 44 that holds a shaft part 6 of the incision electrode 4 of the treatment part 3.

The holding part 44 has the shaft extension part 45 and a spring part 46. The shaft extension part 45 is coupled with a proximal end of the shaft part 6 of the incision electrode 4. The spring part 46 couples a proximal end of the shaft extension part 45 and the proximal end support part 43. The terminal 18 of the switching mechanism 16 is disposed on an outer circumferential surface of a distal end of the shaft extension part 45. Thereby, when the terminal 17 and the terminal 18 of the switching mechanism 16 are in contact with each other, a high-frequency current can be supplied to the incision electrode 4 through the power-supplying cable 26.

The spring part 46 biases the shaft extension part 45 against the proximal end support part 43 toward the distal end side thereof to maintain the contact between the terminal 17 and the terminal 18.

An attachment/detachment mechanism (not shown) keeping the terminal 17 and the terminal 18 in contact with each other may be provided in the medical instrument 1A according to the present embodiment. This attachment/detachment mechanism may be configured such that the terminal 17 and the terminal 18 are separated by a spring force of the flexible spring section 42a.

Next, an operation of the medical instrument 1A according to the present embodiment will be described.

In the present embodiment, for example, when the medical instrument 1A according to the present embodiment is inserted into the channel 101 of the endoscope 100, a traction force of the first wire 24 and a traction force of the second wire 25 are released. Thereby, the flexible spring part 42 is in an expanded state, and the incision electrode 4 is stored in the flexible spring part 42. That is, in the present embodiment, in place of the storage part 19 described in the first embodiment, a storage part 47 is formed in the flexible spring part 42 (see Fig. 8).

During use of the medical instrument 1A, when treatment using the incision electrode 4 is performed, the first wire 24 and the second wire 25 are pulled toward the proximal end sides thereof at the same time. Thereby, the flexible spring part 42 is contracted (see Fig. 9). At this time, as the flexible spring section 42a that is easily contracted is contracted at first, the element wires constituting the flexible spring section 42a come into close contact with one another. It is not essential for the element wires of the flexible spring section 42a to come into close contact. In this state, the incision electrode 4 is fed from the distal end of the distal end support part 41. Since the terminal 18 disposed at the shaft extension part 45 abuts the terminal 17 disposed at the distal end support part 41, a high-frequency current is supplied to the incision electrode 4.

As illustrated in Fig. 10, in a state in which the incision electrode 4 is fed from the distal end of the distal end support part 41, if the first wire 24 and the second wire 25 are pulled toward the proximal end sides thereof at the same time, the incision electrode 4 can be displaced toward the proximal end side thereof substantially in parallel to the wires. When the traction forces of the first and second wires 24 and 25 toward the proximal end sides are released, the incision electrode 4 returns to its original position due to the action of the spring part 46.

In the state in which the incision electrode 4 is fed from the distal end of the distal end support part 41, if any one of the first wire 24 and the second wire 25 is pulled toward the proximal end side thereof, the rigid spring section 42b is bent depending on the pulled wire. At this time, the flexible spring section 42a may be bent along with the rigid spring section 42b.

As described above, in the present embodiment, the storage part 47 storing the incision electrode 4 can be formed in the expansion pipe part 40 by the flexible spring part 42 and the first and second wires 24 and 25 that bend the flexible spring part 42. Thereby, when treatment of the treatment target portion is performed using the treatment part 3, both the mechanism that optimizes the direction and orientation of the treatment part 3 and the mechanism that stores the treatment part 3 in the storage part 47 can be used in common. Therefore, in comparison with a medical instrument that has a separate constitution specialized for storing the treatment part 3, the medical instrument 1A can be miniaturized. Thereby, it is possible to easily pass the insertion part 2 of the medical instrument 1A into the channel 101 that is in a curved state.

### (Third embodiment)

Next, a medical system according to a third embodiment of the present invention will be described.

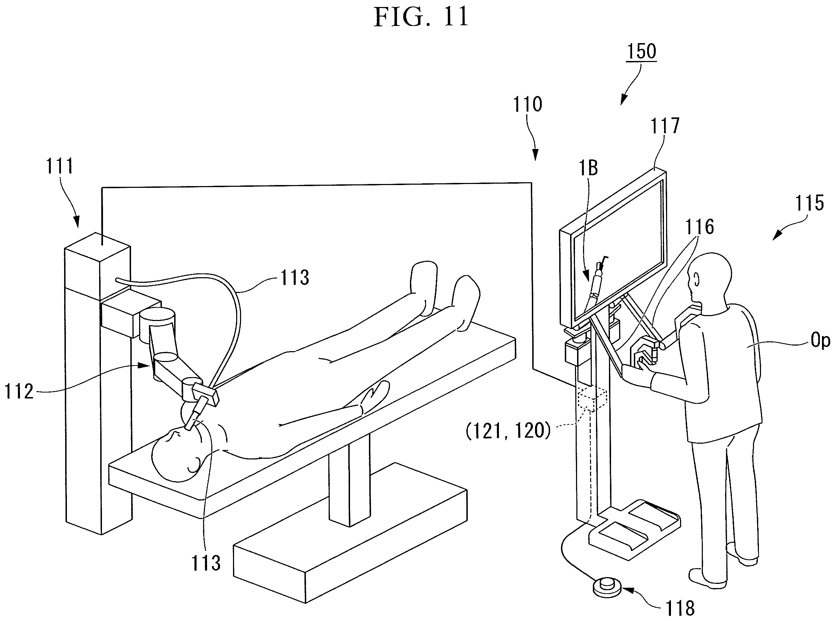

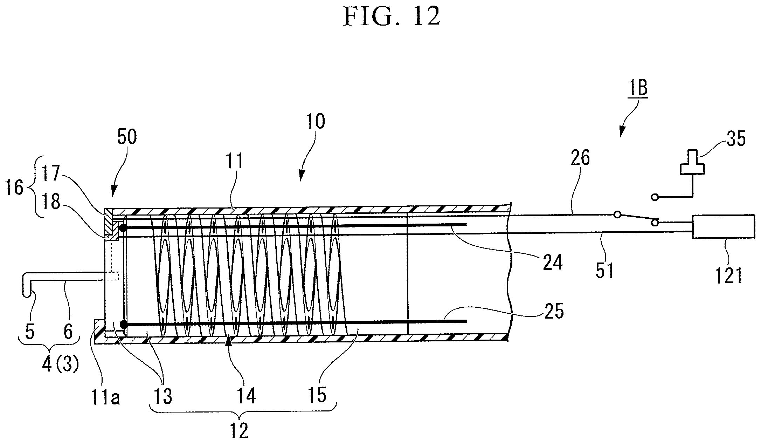

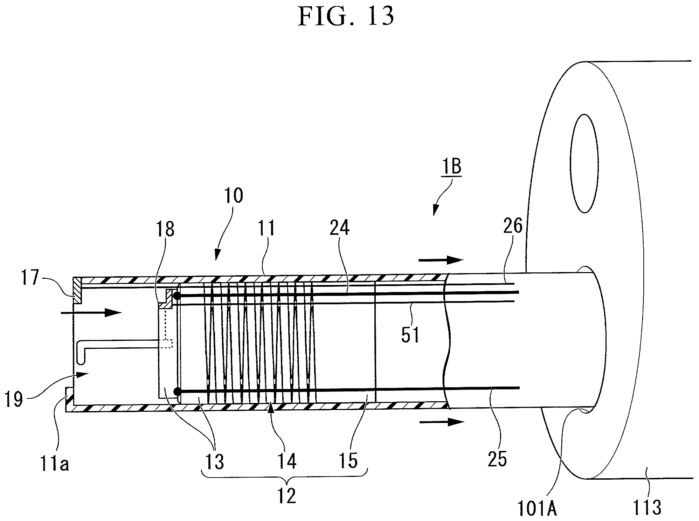

Fig. 11 is a schematic overall view of a medical system according to the third embodiment of the present invention. Fig. 12 is a schematic partial sectional view of a medical instrument according to the third embodiment of the present invention, wherein the medical instrument is provided in the medical system. Fig. 13 is a view for describing an operation of the medical system.

As illustrated in Fig. 11, the medical system 150 according to the present embodiment includes a manipulator device 110 and a medical instrument 1B. The manipulator device 110 is configured for an operator Op who performs an operation on a patient to operate on the patient. The medical instrument 1B is mounted on the manipulator device 110.

The manipulator device 110 includes a slave manipulator 111, a master manipulator 115, and a controller 120. The medical instrument 1B is mounted in the slave manipulator 111. The master manipulator 115 is electrically connected to the slave manipulator 111, and sends a manipulating instruction to the slave manipulator 111. The controller 120 controls the entire medical system 150.

The slave manipulator 111 has a slave arm 112, an actuator (not shown), and a sensor (not shown). At least the aforementioned medical instrument 1B is mounted in the slave arm 112. The actuator operates the slave arm 112. The sensor detects a position of the slave arm 112. The slave manipulator 111 receives the manipulating instruction from the master manipulator 115. The slave manipulator 111 operates the slave arm 112 and the medical instrument 1B in accordance with the manipulating instruction. That is, in the present embodiment, the slave manipulator 111 is connected to at least the driving force generator 32 of the medical instrument 1B. The slave manipulator 111 can operate the driving force generator 32 in response to the manipulating instruction from the master manipulator 115. The slave arm 112 according to the present embodiment has a flexible elongated member 113 inserted into a human body. The elongated member 113 is formed with a channel 101A through which the insertion part 2 of the medical instrument 1B is inserted (see Fig. 13). An imaging mechanism (not shown) for observing the treatment target portion is provided at a distal end of the elongated member 113 of the slave arm 112. This imaging mechanism acquires an image of the treatment target portion.

The master manipulator 115 has master arms 116 and a display unit 117. The master arms 116 are configured such that the operator Op can operate the medical instrument 1B by holding the master arms 116 in his/her hand and operating the master arms 116. The display unit 117 displays the image acquired by the imaging mechanism provided at the elongated member 113 of the slave arm 112. The master manipulator 115 according to the present embodiment has a storage state control switch 118 that sends a trigger signal to the controller 120. The trigger signal is used in the medical instrument 1B to store the treatment part 3 in the storage part 19 or to feed the treatment part 3 from the storage part 19.

The controller 120 outputs signals that operate the slave manipulator 111 based on the manipulating instruction and the trigger signal sent from the master manipulator 115 by the master arms 116 of the master manipulator 115 being operated, to the slave manipulator 111.

The controller 120 outputs a signal that operates the driving force generator 32 of the medical instrument 1B. The controller 120 has a determination unit 121 that determines a state in which the treatment part 3 is inside the storage part 19 and a state in which the treatment part 3 is outside the storage part 19.

The medical instrument 1B has a signal line 51 to detect conduction between the terminal 17 and the terminal 18 of the switching mechanism 16. A distal end of the signal line 51 is connected to the terminal 18 of the switching mechanism 16. A proximal end of the signal line 51 is connected to the determination unit 121. The power-supplying cable 26 can be electrically connected to the determination unit 121. That is, in the present embodiment, when the terminal 17 and the terminal 18 of the switching mechanism 16 are connected, the power-supplying cable 26 and the signal line 51 are connected. When the terminal 17 and the terminal 18 of the switching mechanism 16 are separated from each other, the power-supplying cable 26 and the signal line 51 are insulated. That is, in the present embodiment, the medical instrument 1B is provided with a switch part (switch mechanism) 50 having the power-supplying cable 26, the terminal 17, the terminal 18, and the signal line 51.

The driving force generator 32 (see Fig. 1) of the medical instrument 1B is connected to the controller 120 of the manipulator device 110. The driving force generator 32 is configured to pull the first and second wires 24 and 25 in response to the manipulating instruction based on manipulation input from the master arms 116, and in response to the trigger signal sent from the storage state control switch 118 of the master manipulator 115.

Next, detailed constitutions of the determination unit 121 and the controller 120 in the medical system 150 according to the present embodiment will be described along with an operation of the medical system 150.

In the present embodiment, as the storage state control switch 118 of the master manipulator 115 is pushed by the operator Op, insertion or removal of the treatment part 3 into or from the storage part 19 is initiated based on the trigger signal sent from the storage state control switch 118.

The determination unit 121 detects whether or not the terminal 17 and the terminal 18 of the switching mechanism 16 are connected. That is, the determination unit 121 discriminates and determines the state in which the terminal 17 and the terminal 18 are connected (i.e., the state in which the treatment part 3 is outside the storage part 19, see Fig. 12) and the state in which the terminal 17 and the terminal 18 are not connected (i.e., the state in which the treatment part 3 is inside the storage part 19, see Fig. 13).

If the terminal 17 and the terminal 18 are connected, the determination unit 121 informs the controller 120 of the state in which the treatment part 3 is out of the storage part 19 and treatment on the treatment target portion is possible. If the terminal 17 and the terminal 18 are not connected, the determination unit 121 informs the controller 120 of the state in which the treatment part 3 is stored in the storage part 19 and treatment is impossible. When the terminal 17 and the terminal 18 are not connected, the determination unit 121 may be configured to inform the controller 120 that the treatment part 3 is stored in the storage part 19 and the medical instrument 1B can be replaced.

In the case of the state in which treatment is possible based on a result of the determination by the determination unit 121, the controller 120 causes the display unit 117 to display a status that manipulation input using the master arms 116 of the master manipulator 115 is possible. The controller 120 transmits movement of the master arms 116 of the master manipulator 115 to the slave manipulator 111 as a manipulating instruction to the slave manipulator 111. In the case of the state in which treatment is impossible or the medical instrument 1B can be replaced based on a result of the determination by the determination unit 121, the controller 120 causes the display unit 117 to display a status that manipulation input using the master arms 116 of the master manipulator 115 is impossible and the replacement of the medical instrument 1B is possible. The controller 120 invalidates the manipulating instruction based on the movement of the master arms 116 of the master manipulator 115. The operator Op of the medical system 150 can replace the medical instrument 1B with a medical instrument that is similar to the medical instrument 1B according to the present embodiment but is different in the constitution of, for instance, the treatment part 3, or a spare medical instrument that is the same as the medical instrument 1B according to the present embodiment, for example, manually.

That is, in the present embodiment, the medical system 150 can switch a treatment mode in which treatment using the treatment part 3 is possible and an instrument replacement mode in which the replacement of the medical instrument is possible.

In the medical instrument 1B that is newly inserted into the channel 101A of the elongated member 113 (see Fig. 13), the treatment part 3 is stored in the storage part 19. After the medical instrument 1B that is newly inserted into the channel 101A is securely inserted into the channel 101A, the operator Op pushes down the storage state control switch 118, and thereby the treatment part 3 of the medical instrument 1B is fed from the storage part 19. Thereby, treatment using the treatment part 3 is possible.

According to the medical system 150 according to the present embodiment, the switch part 50 of the medical instrument 1B allows the determination unit 121 to determine whether or not the treatment part 3 is outside the storage part 19. Since the determination unit 121 determines whether the treatment part 3 is stored in the storage part 19 or outside the storage part 19, the operator Op can reliably understand that the treatment part 3 is stored in the storage part 19 without relying on the image obtained by imaging the treatment target portion.

In the present embodiment, it is possible to insert or remove the treatment part 3 into or from the storage part 19 using the master manipulator 115.

Although preferred embodiments of the present invention have been described, the present invention is not limited to these embodiments. Additions, omissions, substitutions, and other modifications of the constitution are possible without departing from the scope of the present invention. The present invention is not limited by the above description, but is only limited by the appended claims.

### Industrial Applicability

According to the medical instrument described above, both the mechanism that optimizes the direction and orientation of the treatment part and the mechanism that displaces the treatment part in order to store the treatment part in the storage part can be used in common. For this reason, in comparison with a medical instrument that has a separate constitution specialized for storing the treatment part, the medical instrument can be miniaturized. Thereby, it is possible to easily pass the insertion part of the medical instrument into a channel that is in a curved state.

### Reference Signs List

1, 1A, 1B: medical instrument
2: insertion part
3: treatment part
10: joint part
19, 47: storage part
21: driving force transmission part
30: driving mechanism
32: driving force generator
50: switch part (switch mechanism)
111: slave manipulator
115: master manipulator
120: controller
121: determination unit

## Claims

1. A medical instrument (1, 1A, 1B) comprising:
an insertion part (2) configured to be inserted into a human body; and
a driving mechanism (30) coupled to the insertion part (2),
wherein the insertion part (2) has:
a treatment part (3) configured to perform treatment on a treatment target portion;
a joint part (10) configured to support the treatment part (3) and to be capable of changing a direction of the treatment part (3); and
a driving force transmission part (21) connected to the joint part (10) and configured to transmit a driving force that changes the direction of the treatment part (3) to the joint part (10),
the driving mechanism (30) has a driving force generator (32) that is connected to the driving force transmission part (21) and is configured to generate the driving force, and
a storage part (19, 47) configured to store the treatment part (3) in the joint part (10) by the driving force transmitted from the driving force generator (32) to the joint part (10) via the driving force transmission part (21) is formed at the joint part (10),
**characterized in that**
the joint part (10) has a flexible spring part (14, 42) that is bendably configured to change the direction of the treatment part (3) and has a substantially cylindrical shape;
the driving force transmission part (21) has a plurality of linear members (24, 25) that are provided corresponding to a bending direction in bending movement of the joint part (10) and are fixed to a distal end of the flexible spring part (14, 42);
the flexible spring part (14, 42) is bent by at least one of the plurality of linear members (24, 25) being pulled; and
at least two of the plurality of linear members (24, 25) are uniformly pulled or pressed such that the distal end of the flexible spring part (14, 42) advances or retracts in a center axis direction of the flexible spring part (14, 42), and thereby the storage part (19, 47) is formed adjacent to the distal end of the flexible spring part (14, 42).

2. The medical instrument (1, 1A, 1B) according to Claim 1, further comprising a cladding tube part (11) configured to cover an outer surface of the flexible spring part (14, 42),
wherein the distal end of the flexible spring part (14, 42) is retracted toward a proximal end side of the flexible spring part (14, 42) by the plurality of linear members (24, 25), and thereby the storage part (19, 47) is formed in the cladding tube part (11).

3. The medical instrument (1, 1A, 1B) according to Claim 1, wherein:
the treatment part (3) is inserted into the flexible spring part (14, 42); and
the distal end of the flexible spring part (14, 42) is advanced toward a distal end side of the flexible spring part (14, 42) by the plurality of linear members (24, 25), and thereby the storage part (19, 47) is formed in the flexible spring part (14, 42).

4. The medical instrument (1, 1A, 1B) according to any one of Claims 1 to 3, wherein:
the treatment part (3) has an incision electrode (4) configured to receive supply of a high-frequency current to incise biological tissue;
the joint part (10) has a switching mechanism (50) configured to switch a conductive state of the high-frequency current for the incision electrode (4);
the insertion part (2) has a power-supplying cable (26) that is connected to the switching mechanism (50) and is configured such that the high-frequency current is applied;
the driving mechanism (30) has a plug (35) that is connected to the power-supplying cable (26) and is configured to be connectable to a high-frequency power supply; and
the switching mechanism (50) interrupts conduction between the power-supplying cable (26) and the incision electrode (4) when the incision electrode (4) is inside the storage part (19, 47), and connects the power-supplying cable (26) and the incision electrode (4) when the incision electrode (4) is outside the storage part (19, 47).

5. A medical system comprising:
the medical instrument (1B) according to any one of Claims 1 to 4;
a master manipulator (115) configured to receive a manipulation input from an operator (Op);
a controller (120) connected to the master manipulator (115);
a slave manipulator (111) connected to the controller (120) and the driving mechanism (30); and
a determination unit (121) provided in the controller (120) and configured to determine a state in which the treatment part (3) is inside the storage part (19, 47) and a state in which the treatment part (3) is outside the storage part (19, 47),
wherein the medical instrument (1B) has a switch mechanism (50) configured to cause the determination unit (121) to determine the state in which the treatment part (3) is inside the storage part (19, 47) and the state in which the treatment part (3) is outside the storage part (19, 47).

## Patentansprüche

1. Medizinisches Instrument (1, 1A, 1B), umfassend:
ein Einführteil (2), das dazu ausgelegt ist, in einen menschlichen Körper eingeführt zu werden; und
einen Antriebsmechanismus (30), der an das Einführteil (2) gekoppelt ist,
wobei das Einführteil (2) aufweist:
ein Behandlungsteil (3), das dazu ausgelegt ist, eine Behandlung an einem Behandlungszielabschnitt durchzuführen;
ein Gelenkteil (10), das dazu ausgelegt ist, den Behandlungsteil (3) zu unterstützen und eine Richtung des Behandlungsteils (3) ändern zu können; und
ein Antriebskraftsendeteil (21), das mit dem Gelenkteil (10) verbunden und dazu ausgelegt ist, eine Antriebskraft, die die Richtung des Behandlungsteils (3) ändert, an das Gelenkteil (10) zu senden,
der Antriebsmechanismus (30) einen Antriebskraftgenerator (32) aufweist, der mit dem Antriebskraftsendeteil (21) verbunden und dazu ausgelegt ist, die Antriebskraft zu erzeugen, und
ein Lagerteil (19, 47), das dazu ausgelegt ist, das Behandlungsteil (3) in dem Gelenkteil (10) durch die Antriebskraft, die von dem Antriebskraftgenerator (32) über das Antriebskraftsendeteil (21) an das Gelenkteil (10) gesendet wurde, zu lagern, ist an dem Gelenkteil (10) gebildet,
**dadurch gekennzeichnet, dass**
das Gelenkteil (10) ein Biegefederteil (14, 42) aufweist, das biegbar ausgelegt ist, um die Richtung des Behandlungsteils (3) zu ändern, und eine im Wesentlichen zylindrische Form aufweist;
das Antriebskraftsendeteil (21) eine Mehrzahl linearer Elemente (24, 25) aufweist, die entsprechend einer Biegerichtung in Biegebewegung des Gelenkteils (10) vorgesehen und an einem distalen Ende des Biegefederteils (14, 42) fixiert sind;
das Biegefederteil (14, 42) gebogen wird, indem mindestens eins der Mehrzahl linearer Elemente (24, 25) gezogen wird; und
mindestens zwei der Mehrzahl linearer Elemente (24, 25) gleichmäßig derart gezogen oder gedrückt werden, dass das distale Ende des Biegefederteils (14, 42) in einer Mittelachsenrichtung des Biegefederteils (14, 42) aus- oder zurückfährt und dadurch das Lagerteil (19, 47) angrenzend an das distale Ende des Biegefederteils (14, 42) gebildet wird.

2. Medizinisches Instrument (1, 1A, 1B) nach Anspruch 1, das ferner ein Hüllrohrteil (11) umfasst, das dazu ausgelegt ist, eine Außenfläche des Biegefederteils (14, 42) abzudecken,
wobei das distale Ende des Biegefederteils (14, 42) in Richtung einer Proximalendseite des Biegefederteils (14, 42) durch die Mehrzahl linearer Elemente (24, 25) zurückgefahren und dadurch das Speicherteil (19, 47) in dem Hüllrohrteil (11) gebildet wird.

3. Medizinisches Instrument (1, 1A, 1B) nach Anspruch 1, wobei:
das Behandlungsteil (3) in das Biegefederteil (14, 42) eingeführt wird; und
das distale Ende des Biegefederteils (14, 42) in Richtung einer Distalendseite des Biegefederteils (14, 42) durch die Mehrzahl linearer Elemente (24, 25) ausgefahren und dadurch das Speicherteil (19, 47) in dem Biegefederteil (14, 42) gebildet wird.

4. Medizinisches Instrument (1, 1A, 1B) nach einem der Ansprüche 1 bis 3, wobei:
das Behandlungsteil (3) eine Inzisionselektrode (4) aufweist, die dazu ausgelegt ist, eine Lieferung eines Hochfrequenzstroms zu empfangen, um biologisches Gewebe einzuschneiden;
das Gelenkteil (10) einen Schaltmechanismus (50) aufweist, der dazu ausgelegt ist, einen leitenden Zustand des Hochfrequenzstroms für die Inzisionselektrode (4) zu schalten;
das Einführteil (2) ein Stromkabel (26) aufweist, das mit dem Schaltmechanismus (50) verbunden und derart ausgelegt ist, dass der Hochfrequenzstrom angelegt wird,
der Antriebsmechanismus (30) einen Stecker (35) aufweist, der mit dem Stromkabel (26) verbunden und dazu ausgelegt ist, mit einer Hochfrequenzstromversorgung verbunden zu sein; und
der Schaltmechanismus (50) die Leitung zwischen dem Stromkabel (26) und der Inzisionselektrode (4) unterbricht, wenn sich die Inzisionselektrode (4) im Innern des Speicherteils (19, 47) befindet, und das Stromkabel (26) und die Inzisionselektrode (4) verbindet, wenn sich die Inzisionselektrode (4) außerhalb des Speicherteils (19, 47) befindet.

5. Medizinisches System, umfassend:
das medizinische Instrument (1B) nach einem der Ansprüche 1 bis 4;
einen Master-Manipulator (115), der dazu ausgelegt ist, eine Manipulationseingabe von einem Bediener (Op) zu empfangen;
eine Steuereinheit (120), die mit dem Mastermanipulator (115) verbunden ist;
einen Slave-Manipulator (111), der mit der Steuereinheit (120) und dem Antriebsmechanismus (30) verbunden ist; und
eine Bestimmungseinheit (121), die in der Steuereinheit (120) vorgesehen und dazu ausgelegt ist, einen Zustand zu bestimmen, in dem sich das Behandlungsteil (3) im Innern des Speicherteils (19, 47) befindet, und einen Zustand, in dem sich das Behandlungsteil (3) außerhalb des Speicherteils (19, 47) befindet,
wobei das medizinische Instrument (1B) einen Schaltmechanismus (50) aufweist, der dazu ausgelegt ist, zu bewirken, dass die Bestimmungseinheit (121) den Zustand bestimmt, in dem sich das Behandlungsteil (3) im Innern des Speicherteils (19, 47) befindet, und den Zustand, in dem sich das Behandlungsteil (3) außerhalb des Speicherteils (19, 47) befindet.

## Revendications

1. Instrument médical (1, 1A, 1B) comprenant :
une partie d'insertion (2) configurée pour être insérée dans un corps humain ; et
un mécanisme d'entraînement (30) couplé à la partie d'insertion (2),
la partie d'insertion (2) ayant :
une partie de traitement (3) configurée pour réaliser un traitement sur une partie cible de traitement ;
une partie articulation (10) configurée pour supporter la partie de traitement (3) et être capable de changer une direction de la partie de traitement (3) ; et
une partie de transmission de force d'entraînement (21) reliée à la partie articulation (10) et configurée pour transmettre une force d'entraînement qui change la direction de la partie de traitement (3) vers la partie articulation (10),
le mécanisme d'entraînement (30) ayant un générateur de force d'entraînement (32) qui est relié à la partie de transmission de force d'entraînement (21) et qui est configuré pour générer la force d'entraînement, et
une partie de stockage (19, 47), configurée pour stocker la partie de traitement (3) dans la partie articulation (10) par la force d'entraînement transmise du générateur de force d'entraînement (32) à la partie articulation (10) par l'intermédiaire de la partie de transmission de force d'entraînement (21), étant formée au niveau de la partie articulation (10),
**caractérisé par le fait que**
la partie articulation (10) a une partie ressort souple (14, 42) qui est configurée de manière pliable pour changer la direction de la partie de traitement (3) et a une forme sensiblement cylindrique ;
la partie de transmission de force d'entraînement (21) a une pluralité d'éléments linéaires (24, 25) qui sont disposés en correspondance d'une direction de courbure en mouvement de courbure de la partie articulation (10) et sont fixés à une extrémité distale de la partie ressort souple (14, 42) ;
la partie ressort souple (14, 42) est courbée par au moins un parmi la pluralité d'éléments linéaires (24, 25) qui est tiré ; et
au moins deux parmi la pluralité d'éléments linéaires (24, 25) sont tirés ou pressés de manière uniforme de telle sorte que l'extrémité distale de la partie ressort souple (14, 42) avance ou se rétracte dans une direction d'axe central de la partie ressort souple (14, 42) et, de ce fait, la partie de stockage (19, 47) est formée adjacente à l'extrémité distale de la partie ressort souple (14, 42).

2. Instrument médical (1, 1A, 1B) selon la revendication 1, comprenant en outre une partie tube de gainage (11) configurée pour recouvrir une surface externe de la partie ressort souple (14, 42),
l'extrémité distale de la partie ressort souple (14, 42) étant rétractée vers un côté d'extrémité proximale de la partie ressort souple (14, 42) par la pluralité d'éléments linéaires (24, 25) et, de ce fait, la partie de stockage (19, 47) étant formée dans la partie de tube de gainage (11).

3. Instrument médical (1, 1A, 1B) selon la revendication 1, dans lequel :
la partie de traitement (3) est insérée dans la partie ressort souple (14, 42) ; et
l'extrémité distale de la partie ressort souple (14, 42) est avancée vers un côté d'extrémité distale de la partie ressort souple (14, 42) par la pluralité d'éléments linéaires (24, 25) et, de ce fait, la partie de stockage (19, 47) est formée dans la partie ressort souple (14, 42).

4. Instrument médical (1, 1A, 1B) selon l'une quelconque des revendications 1 à 3, dans lequel :
la partie de traitement (3) a une électrode d'incision (4) configurée pour recevoir l'alimentation d'un courant à haute fréquence pour inciser un tissu biologique ;
la partie articulation (10) a un mécanisme de commutation (50) configuré pour commuter vers un état conducteur du courant à haute fréquence pour l'électrode d'incision (4) ;
la partie d'insertion (2) a un câble d'alimentation (26) qui est relié au mécanisme de commutation (50) et qui est configuré de telle sorte que le courant à haute fréquence est appliqué ;
le mécanisme d'entraînement (30) a une fiche (35) qui est reliée au câble d'alimentation (26) et qui est configurée pour être apte à être reliée à une alimentation électrique à haute fréquence ; et
le mécanisme de commutation (50) interrompt la conduction entre le câble d'alimentation (26) et l'électrode d'incision (4) lorsque l'électrode d'incision (4) se trouve à l'intérieur de la partie de stockage (19, 47), et relie le câble d'alimentation (26) et l'électrode d'incision (4) lorsque l'électrode d'incision (4) se trouve à l'extérieur de la partie de stockage (19, 47).

5. Système médical comprenant :
l'instrument médical (1B) selon l'une quelconque des revendications 1 à 4 ;
un manipulateur maître (115) configuré pour recevoir une entrée de manipulation en provenance d'un opérateur (Op) ;
une unité de commande (120) reliée au manipulateur maître (115) ;
un manipulateur esclave (111) relié à l'unité de commande (120) et au mécanisme d'entraînement (30) ; et
une unité de détermination (121) prévue dans l'unité de commande (120) et
configurée pour déterminer un état dans lequel la partie de traitement (3) se trouve à l'intérieur de la partie de stockage (19, 47) et un état dans lequel la partie de traitement (3) se trouve à l'extérieur de la partie de stockage (19, 47), l'instrument médical (1B) ayant un mécanisme de commutation (50) configuré pour amener l'unité de détermination (121) à déterminer l'état dans lequel la partie de traitement (3) se trouve à l'intérieur de la partie de stockage (19, 47) et l'état dans lequel la partie de traitement (3) se trouve à l'extérieur de la partie de stockage (19, 47).
